# EUROPEAN PATENT APPLICATION

(11) **EP 4 400 506 A1**
(43) Date of publication of application: **17.07.2024**
(21) Application number: 22866379.5
(22) Date of filing: 19.08.2022
(51) Int. Cl.: C07J 5/00, C07J 7/00, C07J 21/00, C07J 31/00, C07J 51/00

(54) **INTERMEDIATE COMPOUND AND PREPARATION METHOD THEREFOR AND APPLICATION THEREOF**

(30) Priority: 08.09.2021 CN 202111049973; 07.12.2021 CN 202111516146
(71) Applicant: Hunan Kyf Pharmaceutical. Co., Ltd., Jinshi, Hunan 415400 (CN)
(72) Inventor: LIU, Xirong, Changsha, Hunan 410221 (CN); TANG, Jie, Changsha, Hunan 410221 (CN); LI, Kai, Changsha, Hunan 410221 (CN); GAN, Jie, Changsha, Hunan 410221 (CN)
(74) Representative: Vial, Lionel
(86) International application number: PCT/CN2022/113559
(87) International publication number: WO 2023/035906

(57) **Abstract**

The present application provides an intermediate compound and a preparation method therefor and an application thereof. The intermediate compound has the following structural formula (I). By using the intermediate compound of the present application, dedrogesterone can be conveniently synthesized only by performing AB ring double bond construction and side chain modification, the total yield is high, and the route is short.

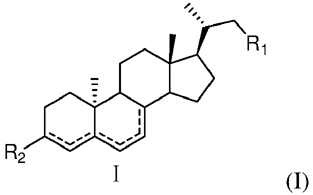

## Description

### FIELD

The present application relates to the field of chemical pharmaceuticals, and in particular to an intermediate compound, a preparation method and an application thereof.

### BACKGROUND

Dydrogesterone, also known as dehydroprogesterone, has a chemical name of 9β,10α-pregna-4,6-diene-3,20-dione. Its CAS number is 152-62-5 and its chemical formula is as follows:

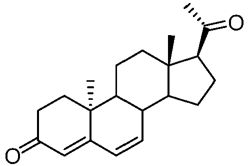

The parent compound of dydrogesterone is pregnane, which has the following skeletal structure comprised of four rings, labeled A, B, C, and D from left to right, with carbon numbers 1-21 designated as C-1, C-2, and so on below.

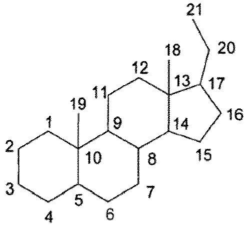

Dydrogesterone is widely used to prevent miscarriage and also to treat various diseases caused by insufficient endogenous progesterone, such as dysmenorrhea, endometriosis, secondary amenorrhea, irregular menstrual cycles, dysfunctional uterine bleeding, premenstrual syndrome, threatened or recurrent miscarriage due to progesterone deficiency, and infertility due to luteal deficiency.

Currently, some synthesis routes begin with ergosterol, which is photochemically synthesized into a 10α configuration intermediate. This intermediate then undergoes Oppenauer oxidation, double bond migration, ozone oxidation, enamination, and finally oxidation to produce dydrogesterone. However, the photoconversion process has a low conversion rate and is difficult in separation. In addition, this process requires ozone oxidation, which poses safety risks and produces many by-products. Another synthetic route involving a 4-step reaction using lumisterol-4,7,22-trien-3-one as the raw material is not feasible for industrialization due to low yield in each step and difficulty in obtaining starting materials. Other synthetic routes use trans-progesterone as the raw material and tetrachlorobenzoquinone as the oxidant. While such synthetic routes are concise, they require the synthesis of trans-progesterone, a raw material that does not occur naturally. Currently, synthesizing this material is challenging and there are no existing industrial products, making industrial production unfeasible at this time.

### SUMMARY

One of the purposes of the present application is to provide a production process for synthesizing dydrogesterone on an industrial scale, with readily available raw materials, high overall yield, and scalability to industrial production.

One aspect of the application provides an intermediate compound having a structural formula of 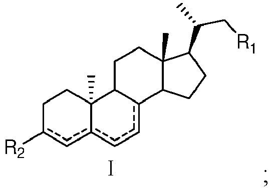
wherein, R₁ is halogen or OR₃;
R₂ is selected from the group consisting of =O, -OR₆, and a protected carbonyl group;
R₃ is selected from the group consisting of H, 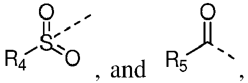 wherein dotted line indicates a connection position with O;
R₄ is selected from the group consisting of a substituted or unsubstituted C1-C6 straight or branched chain alkyl group, a phenyl group unsubstituted or substituted by a C1-C6 alkyl group, a hydroxyl group or a halogen atom, a naphthyl group unsubstituted or substituted by a C1-C6 alkyl group, a hydroxyl group or a halogen atom, and a pyridyl group unsubstituted or substituted by a C1-C6 alkyl group, a hydroxyl group or a halogen atom;
R₅ is selected from the group consisting of a substituted or unsubstituted C1-C6 straight or branched chain alkyl group, a phenyl group unsubstituted or substituted by a C1-C6 alkyl group, a hydroxyl group or a halogen atom, a naphthyl group unsubstituted or substituted by a C1-C6 alkyl group, a hydroxyl group or a halogen atom, and a pyridyl group unsubstituted or substituted by a C1-C6 alkyl group, a hydroxyl group or a halogen atom;
R₆ is H or a hydroxyl protecting group; and
in structural formula I, - - - - - - means that the chemical bond is a single bond or a double bond, and when one - - - - - - is a double bond, the adjacent one - - - - - - is a single bond.

In some embodiments of the application, R₄ is a C1-C3 straight or branched chain alkyl group, or a phenyl group unsubstituted or substituted by a C1-C3 alkyl group; and/or R₅ is selected from the group consisting of a C1-C3 straight or branched chain alkyl group, a phenyl group unsubstituted or substituted by a C1-C3 alkyl group, a naphthyl group unsubstituted or substituted by a C1-C3 alkyl group, and a pyridyl group unsubstituted or substituted by a C1-C3 alkyl group; and/or the hydroxyl protecting group is selected from the group consisting of -C(=O)R₇, a C1-C8 alkyl group, and a C1-C8 silyl group; and/or R₇ is a substituted or unsubstituted C1-C6 straight or branched alkyl group; and/or the protected carbonyl group is a ketal; and/or the halogen or the halogen atom is selected from the group consisting of Cl, Br, and I.

In some embodiments of the present application, R₃ is selected from the group consisting of H, 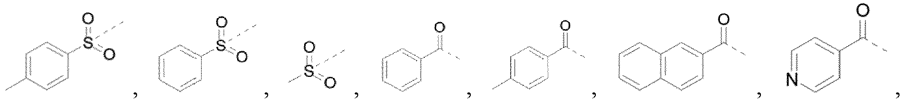 and

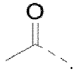

In some embodiments of the present application, the intermediate compound comprises a structural formula selected from the group consisting of: 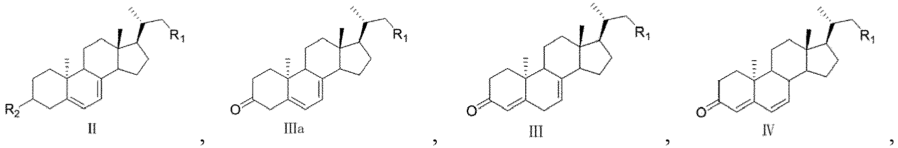 and 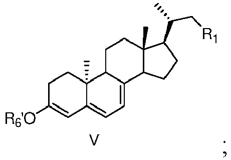 wherein, R₂ is -OR₆ or a protected carbonyl group, and R₆' is a C1-C8 alkyl group.

In some embodiments of the present application, the intermediate compound comprises a structural formula selected from the group consisting of:

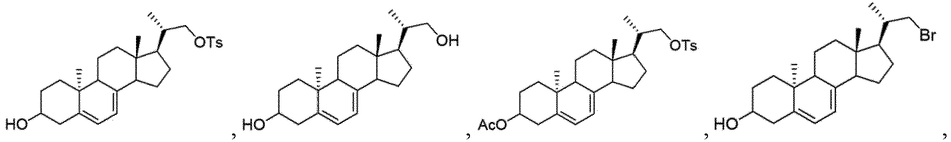

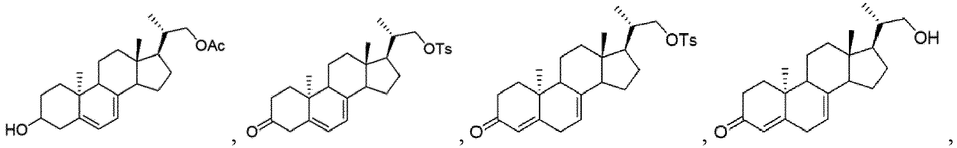

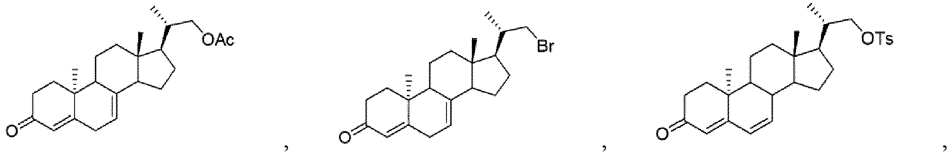

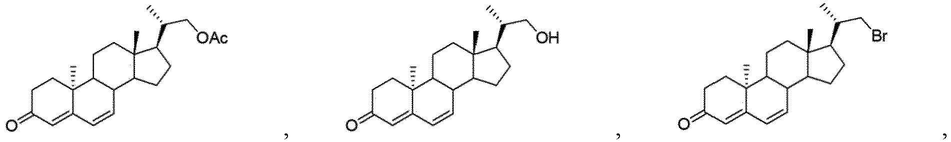

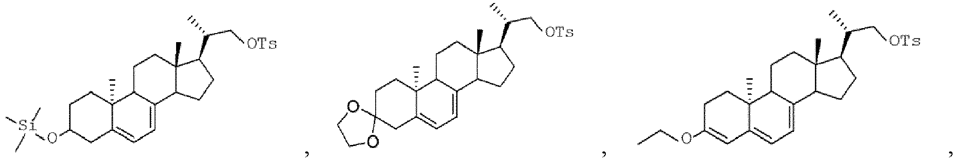

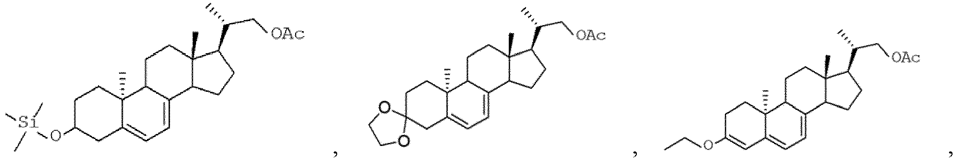

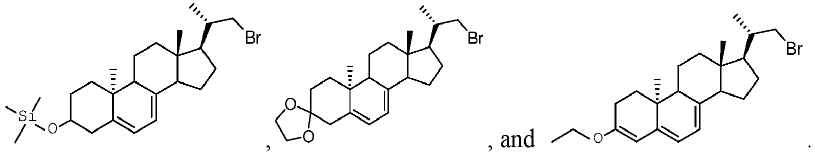

Another aspect of the present application also provides a method for preparing the intermediate compound described in any one of the above, comprising subjecting a compound represented by structural formula IIa to photochemical conversion to switch the methyl group at C-10 from β-configuration to α-configuration to obtain a compound represented by structural formula II;

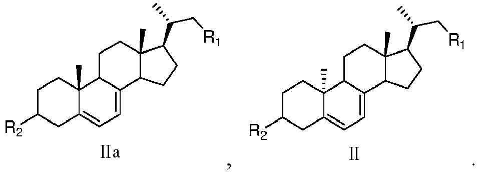

In some embodiments of the present application, the method comprises subjecting the compound represented by structural formula IIa to photochemical conversion under the irradiation of an ultraviolet high-pressure mercury lamp to switch the methyl group at C-10 from β-configuration to α-configuration; wherein ultraviolet light emitted by the ultraviolet high-pressure mercury lamp is filtered by an optical filter liquid for photochemical conversion, and the optical filter liquid comprises Cu²⁺; wherein the photochemical conversion is carried out using an optical filter liquid of a first stage and an optical filter liquid of a second stage, and a concentration of Cu²⁺ in the optical filter liquid of the first stage is less than or equal to a concentration of Cu²⁺ in the optical filter liquid of the second stage.

In some embodiments of the present application, the optical filter liquid of the first stage filters out part or all of the light with a wavelength less than 270 nm, and the optical filter liquid of the second stage filters out part or all of the light with a wavelength less than 300 nm.

In some embodiments of the present application, the concentration of Cu²⁺ in the optical filter liquid of the first stage is 0.1-0.5 wt%, and the concentration of Cu²⁺ in the optical filter liquid of the second stage is 0.5-1.2 wt%. Optionally, the concentration of Cu²⁺ in the optical filter liquid of the first stage is 0.3-0.5 wt%, such as 0.3 wt%, 0.4 wt%, or 0.5 wt%, and the concentration of Cu²⁺ in the optical filter liquid of the second stage is 0.7-1 wt%, such as 0.7 wt%, 0.8wt%, 0.9 wt%, or 1 wt%.

In some embodiments of the present application, R₂ in the compound represented by structural formula IIa is -OR₆; and the compound represented by structural formula II is a compound represented by structural formula IIb;

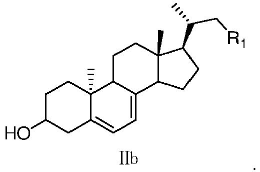

In some embodiments of the present application, the method further comprises subjecting the compound represented by structural formula IIb to oxidation of the hydroxyl group at C-3 and double bond migration at C-5,6 to obtain a compound represented by structural formula III;

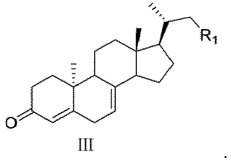

In some embodiments of the present application, the compound represented by structural formula III is converted from the compound represented by structural formula IIb by performing an Oppenauer oxidation reaction to oxidize the hydroxyl group at C-3 to a ketone group and to migrate the double bond from C-5,6 to C-4,5; or
performing oxidation treatment on the compound represented by structural formula lib by using an oxidizing reagent to oxidize the hydroxyl group at C-3 in the compound represented by structural formula IIb to a ketone group, wherein the oxidizing reagent has a structural formula of 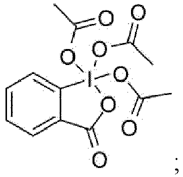 and performing alkaline treatment to migrate the double bond from C-5,6 to C-4,5 to obtain the compound represented by structural formula III.

In some embodiments of the present application, an oxidizing reagent is used for the oxidation treatment, and the oxidizing reagent and the compound represented by structural formula IIb are at a molar ratio of (1.2-1.8): 1.

In some embodiments of the present application, bicarbonate and water are added during the oxidation treatment, and the bicarbonate, the water and the compound represented by structural formula lib are at a molar ratio of (1.5-2.5):(0.8-1.2): 1.

In some embodiments of the present application, an organic amine is used for the alkaline treatment.

In some embodiments of the present application, the method further comprises subjecting the compound represented by structural formula III to double bond migration to obtain a compound represented by structural formula IV;

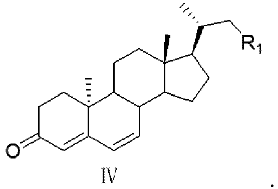

In some embodiments of the present application, the compound represented by structural formula IV is converted from the compound represented by structural formula III by subjecting the compound represented by structural formula III to a protonic acid condition to migrate the double bond from C-7,8 to C-6,7 to obtain the compound represented by structural formula IV; wherein the protonic acid is added in the form of an alcohol solution of hydrogen halide, and the alcohol is selected from the group consisting of ethanol, isopropanol, butanol, ethanediol, and a mixture thereof.

In some embodiments of the present application, the compound represented by structural formula IV is converted from the compound represented by structural formula III by adding the alcohol solution of hydrogen halide to a reaction solvent comprising the compound represented by structural formula III; wherein the alcohol solution of hydrogen halide is added at an amount of 10v-15v (That is, the ratio of the volume of the alcohol solution of hydrogen halide to the mass of the compound is 10 mL-15 mL:1 g. In other words, for 1 g of the compound, the alcohol solution of hydrogen halide is added at an amount of 10 mL-15 mL), a mass percentage of water in the alcohol solution of hydrogen halide is less than 0.2%, and a weight of hydrogen halide accounts for 25wt%-40wt% of a total weight of the alcohol solution of hydrogen halide.

In some embodiments of the present application, the method further comprises adding an antioxidant to the reaction solvent comprising the compound represented by structural formula III, and a mass of the antioxidant accounts for 0.8%-1.2% of a mass of the compound represented by structural formula III.

In some embodiments of the present application, the intermediate compound is compound E; the method further comprises hydrolyzing the compound represented by structural formula IV to obtain compound E; 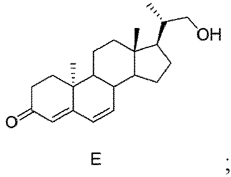 and R₁ is halogen or OR₃ in the compound represented by structural formula IV, wherein R₃ is

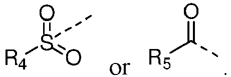

The present application also provides application of the intermediate compound described in any one of the above in the manufacture of dydrogesterone by constructing a ketone group at C-20 of the intermediate compound.

In some embodiments of the present application, the intermediate compound has the following structural formula or the intermediate compound is converted into an intermediate compound with the following structure: 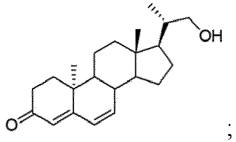 and the ketone group is constructed by:
oxidizing the hydroxyl group at C-21 of the intermediate compound into an aldehyde group to obtain compound F; and
subjecting the aldehyde group of compound F to an enamination reaction and then to oxidization at C-20 into a carbonyl group to obtain dydrogesterone;

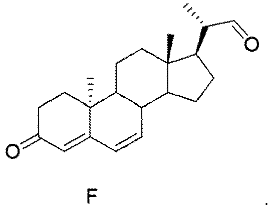

The present application provides a new method for preparing dydrogesterone that is urgently needed in this field. The starting raw material used in this method can be prepared from the fermentation product of phytosterols, which have a wide range of sources and are environmentally friendly. The intermediate compound of the technical solution of the present application can conveniently synthesize dydrogesterone only by constructing the double bonds of rings A and B and modifying the side chain. The method has a high overall yield and a short route, and is a new process for synthesizing dydrogesterone on an industrial scale. The method therefore solves the problems in the prior art such as difficulty in obtaining raw materials, low conversion rate in the photoconversion process, a high amount of by-products, high safety risks, and difficulty in industrial production.

### DETAILED DESCRIPTION

Unless otherwise defined, all technical and scientific terms used herein have the same meanings as commonly understood by those of ordinary skill in the art to which the present disclosure belongs. If there is a conflict, the definition provided in the present application shall prevail. When a trade name appears herein, it is meant to refer to its corresponding commodity or its active ingredient. All patents, published patent applications and publications cited herein are all incorporated herein by reference.

The term "one or more" or similar expressions "at least one" can mean, for example, 1, 2, 3, 4, 5, 6, 7, 8, 9, 10 or more.

The expression m-n used herein refers to the range of m to n, the sub-range composed of each point value therein, and each point value therein. For example, the expression "C1-C6" or "C1-6" covers the range of 1-6 carbon atoms, and should be understood as also covering any sub-range therein and each point value therein, such as C2-C5, C3-C4, C1-C2, C1-C3, C1-C4, C1-C5, C1-C6, C1-C7, etc., and C1, C2, C3, C4, C5, C6, C7, C8, etc.

The term "alkyl" refers to a straight or branched chain saturated aliphatic hydrocarbon group composed of carbon atoms and hydrogen atoms, which is attached to the rest of the molecule through a single bond. "Alkyl" can have 1-8 carbon atoms, that is, "C1-8 alkyl", such as C1-4 alkyl, C1-3 alkyl, C1-2 alkyl, C3 alkyl, C4 alkyl, C1-6 alkyl, and C3-6 alkyl. Non limiting examples of alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or isomers thereof.

The term "silyl" refers to an alkyl group as defined above in which at least one C atom is substituted with a Si atom. The silyl group is attached to the rest of the molecule through a silicon atom. "C1-8 silyl" refers to a silyl group containing 1-8 carbon atoms, in which the alkyl moiety can be straight, branched chain or cyclic. Silyl includes, but is not limited to, trimethylsilyl (TMS), tert-butyldimethylsilyl (TBS, or referred to as TBDMS), dimethylisopropylsilyl (IPDMS), di-tert-butylmethylsilyl (DTBMS), etc.

The compound of the present disclosure may exist in specific geometric or stereoisomeric forms. The present disclosure contemplates all such compounds, including cis and trans isomers, (-)- and (+)- enantiomers, (R)- and (S)-enantiomers, diastereomer, (D)-isomers, (L)-isomers, and racemic mixtures thereof, as well as other mixtures, such as enantiomer- or diastereomer-enriched mixtures, and all of these mixtures fall within the scope of the present disclosure. The purification and separation of such substances can be achieved by standard techniques known in the art.

The following detailed description of the present disclosure is intended to illustrate non-limiting embodiments to make other skilled in the art more fully understand the technical solution of the present disclosure, principles and practical applications thereof, so that other skilled in the art can modify and implement the present disclosure in many ways to optimally adapt it to the requirements of a specific use.

### Intermediate compounds

In an aspect, the embodiments of the present application provide an intermediate compound for application in preparing dydrogesterone, having a structural formula of 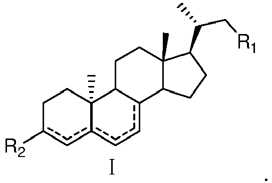 In structural formula I, R₁ is halogen or OR₃; R₂ is selected from the group consisting of =O, -OR₆, and a protected carbonyl group; R₃ is selected from the group consisting of H, 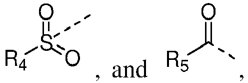 wherein dotted line indicates a connection position with O; R₄ is selected from the group consisting of a substituted or unsubstituted C1-C6 straight or branched chain alkyl group, a phenyl group unsubstituted or substituted by a C1-C6 alkyl group, a hydroxyl group or a halogen atom, a naphthyl group unsubstituted or substituted by a C1-C6 alkyl group, a hydroxyl group or a halogen atom, and a pyridyl group unsubstituted or substituted by a C1-C6 alkyl group, a hydroxyl group or a halogen atom; R₅ is selected from the group consisting of a substituted or unsubstituted C1-C6 straight or branched chain alkyl group, a phenyl group unsubstituted or substituted by a C1-C6 alkyl group, a hydroxyl group or a halogen atom, a naphthyl group unsubstituted or substituted by a C1-C6 alkyl group, a hydroxyl group or a halogen atom, and a pyridyl group unsubstituted or substituted by a C1-C6 alkyl group, a hydroxyl group or a halogen atom; R₆ is H or a hydroxyl protecting group.

In structural formula I, - - - - - - means that the chemical bond is a single bond or a double bond, and when one - - - - - - is a double bond, the adjacent one - - - - - - is a single bond.

R₄ is a C1-C3 straight or branched chain alkyl group, or a phenyl group unsubstituted or substituted by a C1-C3 alkyl group; and/or R₅ is selected from the group consisting of a C1-C3 straight or branched chain alkyl group, a phenyl group unsubstituted or substituted by a C1-C3 alkyl group, a naphthyl group unsubstituted or substituted by a C1-C3 alkyl group, and a pyridyl group unsubstituted or substituted by a C1-C3 alkyl group; and/or the hydroxyl protecting group is selected from the group consisting of -C(=O)R₇, a C1-C8 alkyl group, and a C1-C8 silyl group; and/or R₇ is a substituted or unsubstituted C1-C6 straight or branched alkyl group; and/or the protected carbonyl group is a ketal; and/or the halogen or the halogen atom is selected from the group consisting of Cl, Br, and I.

In some embodiments, R₃ is selected from the group consisting of H,

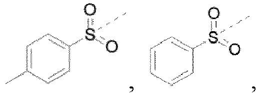

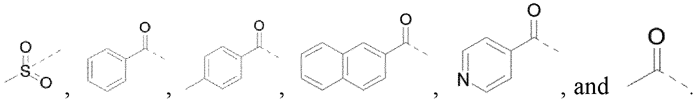

In some embodiments, the intermediate compound comprises a structural formula selected from the group consisting of: 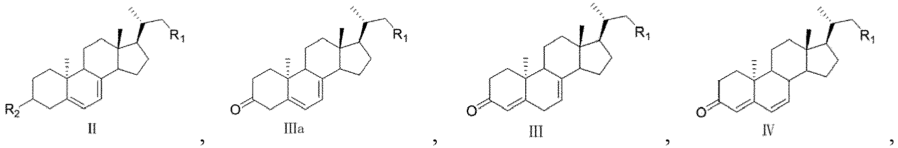 and 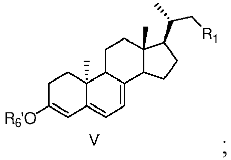 wherein, R₂ is -OR₆ or a protected carbonyl group; R₆ is H or a hydroxyl protecting group, wherein the protected carbonyl group is a ketal; and R₆' is a C1-C8 alkyl group.

As an example, some specific structural formulas of the structural formula I are as follows:

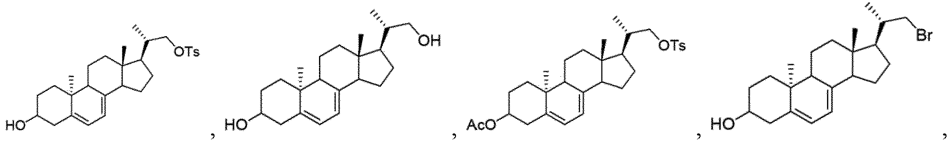

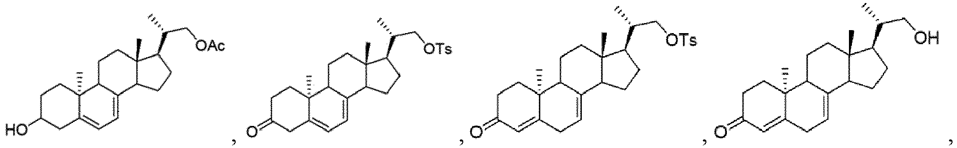

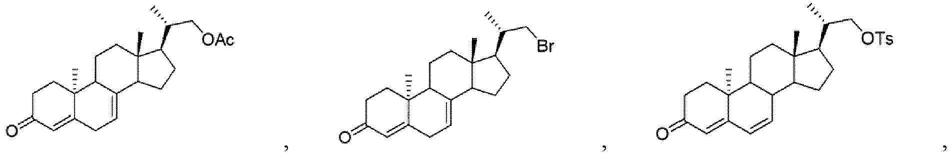

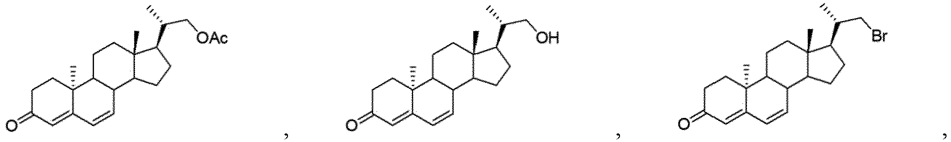

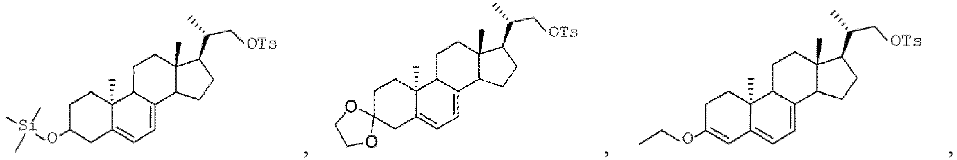

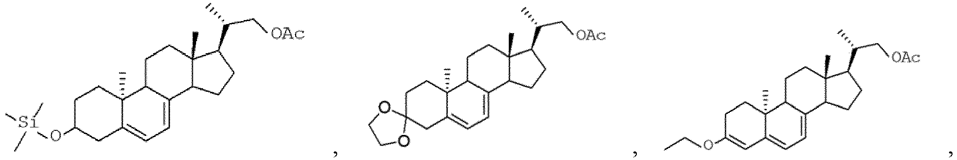

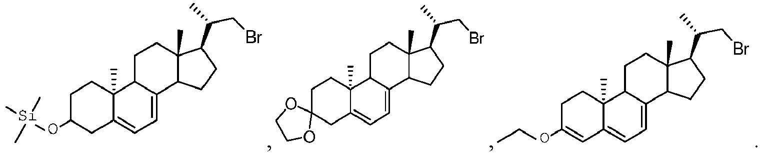

The hydroxyl or carbonyl groups of some of these compounds can be protected by a protecting group. For example: 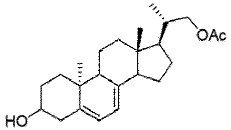 The protected structural formula is 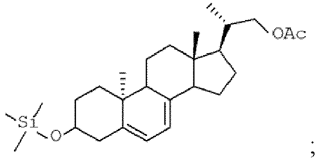 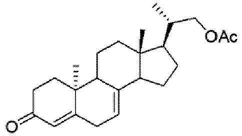 The protected structural formula is 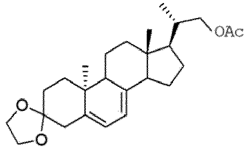 or

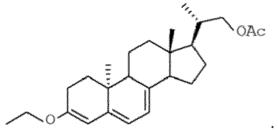

### Methods for preparing intermediate compounds

### (Steps of photochemical conversion)

The embodiments of the present application also provide a method for preparing the intermediate compound described in any of the above. The method comprises subjecting a compound represented by structural formula IIa to photochemical conversion to switch the methyl group at C-10 from β-configuration to α-configuration to obtain a compound represented by structural formula II;

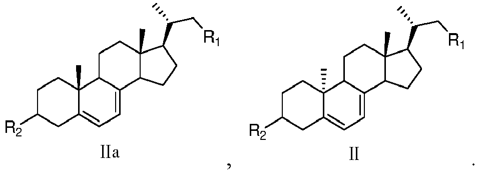

In some embodiments, R₂ is -OR₆. In some embodiments, R₆ is H, the compound represented by structural formula II is a compound represented by structural formula IIb, and the compound represented by structural formula IIb is converted from the compound represented by structural formula IIa through a two-step photochemical conversion reaction;

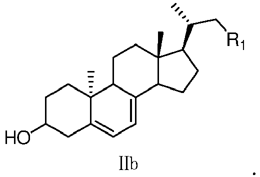

In some embodiments, the two-step photochemical conversion reaction may comprise: subjecting the compound represented by structural formula IIa to irradiation with ultraviolet light of a first wavelength for ring opening as the first step of photochemical conversion reaction; and subjecting the ring-opened compound represented by structural formula IIa to irradiation with ultraviolet light of a second wavelength for ring closing as the second step of photochemical conversion reaction.

Plant sterols can be used as raw materials and fermented by microorganisms of the genus *Mycobacterium* to obtain a fermentation product (see the formula below), and then the compound represented by structural formula IIa can be obtained through chemical synthesis. When R₂ is -OR₆, the compound represented by structural formula IIa can be obtained through shorter steps. Therefore, the raw materials of the embodiments of the present application are readily available.

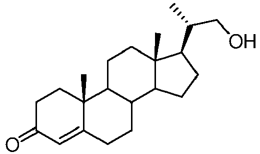

In some embodiments, the reaction solvents of the first step photochemical conversion reaction and the second step photochemical conversion reaction are at least one of methanol, ethanol, n-hexane, petroleum ether, n-heptane, ethyl acetate, tetrahydrofuran, ethanediol, and isopropanol, the reaction temperature is -10°C to 50°C, the first wavelength is 270 nm to 290 nm, and the second wavelength is 300 nm to 330 nm. In some embodiments, a high-pressure mercury lamp or an LED lamp is used to provide ultraviolet light.

LED ultraviolet lamps provide a single wavelength, low power, and limited wavelength selection range. Large-scale photochemical reactions require the integration of a large number of LED lamp beads. The production of light sources is complex and the cost is high. In one embodiment, an ultraviolet high-pressure mercury lamp is used. Using an ultraviolet high-pressure mercury lamp can achieve greater power and reduce costs. However, the ultraviolet high-pressure mercury lamp provide a wide spectral range (the ultraviolet region has a broad spectrum distribution from 250 nm to 370 nm). Mechanistically speaking, the wavelengths required for the two-step photochemical reaction of the present disclosure are 270-300 nm in the first stage and 300-350 nm in the second stage. If the ultraviolet high-pressure mercury lamp is directly used for irradiation during the first stage of ring opening, it will lead to a high proportion of by-product configuration because it contains undesired wavelengths (such as light with wavelengths around 254 nm). Therefore, it is necessary to filter the light from the ultraviolet high-pressure mercury lamp.

A device containing an optical filter liquid is arranged between the reaction system and the light source, so that the light first passes through the optical filter liquid to be filtered, and then irradiates into the reaction system.

Regarding the optical filter liquid, it comprises Cu²⁺ and can be a copper salt, such as copper sulfate, copper chloride, copper acetate, etc., or it can also comprise other substances or impurities that do not affect the filtering effect of Cu²⁺ and are colorless in water. The optical filter liquid can be an aqueous solution, or other colorless solvents that do not affect the filtering effect of Cu²⁺ and can dissolve Cu²⁺.

Regarding the optical filter liquid, after filtering through an aqueous solution of Cu²⁺ with a concentration of 0.1-0.5 wt%, specifically 0.3-0.5 wt%, most of the wavelengths are greater than 270 nm (light with wavelengths below 270 nm is basically filtered out); after filtering through an aqueous solution of Cu²⁺ with a concentration of 0.5-1.2 wt%, specifically 0.7-1 wt%, most of the wavelengths are greater than 300 nm, reaching the wavelength range required for photochemical reactions. That is, the intensity ratio of ultraviolet rays of different wavelengths is adjusted through the optical filter liquid, so that the proportion of favorable wavelengths is higher.

The use of low-concentration optical filter liquid in the first stage of ring opening is conducive to ring opening and conversion to the desired configuration, and reduces damage to raw materials, while ring closing requires a higher concentration of optical filter liquid to enhance the filtration of low wavelengths, which shifts the reaction equilibrium toward ring closing and can thus achieve higher yields.

Photochemical conversion produces multiple products. When R₁ is an aromatic group such as OTs, the photochemical conversion reaction in this step has better conversion rate and selectivity (more than 40% of selectivity), and can achieve better yield (25%); additionally, it is easier to separate the compound represented by structural formula lib from the product, and the post-processing is simple.

When R₁ in the compound represented by structural formula lib is -OR₃ and R₃ is 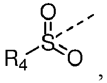 the hydroxyl group at C-3 can be protected first, and then R1 can be converted into an ester group and then deprotected. This can make the esterification product easier to separate.

### (Steps of double bond migration under oxidation and alkaline conditions)

In some embodiments, the method further comprises subjecting the compound represented by structural formula IIb to oxidation of the hydroxyl group at C-3 and double bond migration at C-5,6 to obtain a compound represented by structural formula III;

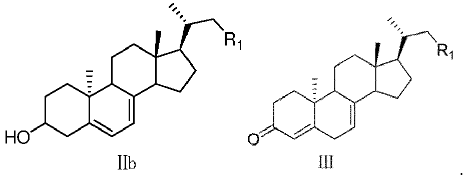

The structure of the compound represented by structural formula IIb has particularities. For example, the conjugated double bond in ring B worsens the structure, the α-configuration methyl group at C-10 changes the solubility of the compound, and the group at C-21 position is different, etc. As a result, this oxidation reaction has higher requirements on the oxidation system. If some commonly used oxidation systems are used, either the target product cannot be obtained or the yield is very low. For example, Swern oxidation can be used to obtain the compound represented by structural formula III, but the yield is very low, impurities are difficult to control and the conditions are harsh.

Oppenauer oxidation reaction can be used to oxidize the hydroxyl group at C-3 to a ketone group and to migrate the double bond from C-5,6 to C-4,5. Oppenauer oxidation (reagents such as aluminum isopropoxide/cyclohexanone) is a high-temperature reaction, and there are high-boiling substances that must be removed at high temperatures. Although Oppenauer oxidation can carry out double bond migration at the same time, the compound represented by structural formula III is unstable under high temperature or strong alkaline conditions, resulting in a low reaction yield (molar yield of about 48%).

In some embodiments, the compound represented by structural formula III is converted from the compound represented by structural formula IIb by: performing oxidation treatment on the compound represented by structural formula IIb to oxidize the hydroxyl group at C-3 in the compound represented by structural formula lib to a ketone group; and performing alkaline treatment to migrate the double bond from C-5,6 to C-4,5 to obtain the compound represented by structural formula III

The oxidizing reagent used in the embodiments of the present application has the following structural formula (Dess-Martin periodinane): 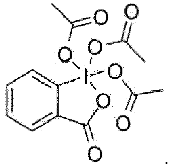 It can perform oxidation reactions at low temperatures (such as -5°C to 25°C, or 5°C to 10°C). The molar ratio of the oxidizing reagent to the compound represented by structural formula lib can be (1.2-1.8):1. In some embodiments, water and bicarbonate (such as sodium bicarbonate, potassium bicarbonate) are also added during the oxidation treatment, which can promote the reaction and increase the conversion rate and yield. The conversion rate can reach 92%, and the molar yield can reach 70%. The molar ratio of the bicarbonate, water and the compound represented by structural formula lib is (1.5-2.5): (0.8-1.2):1. Then an organic base, preferably an amine, such as triethylamine, pyridine, etc., is used for alkaline treatment under mild conditions, to obtain a higher yield of the compound represented by structural formula III. This method avoids the effects of high temperature, long-term concentration, and strong alkali.

### (Steps of double bond migration under acidic conditions)

In some embodiments, the method further comprises subjecting the compound represented by structural formula III to double bond migration to obtain a compound represented by structural formula IV;

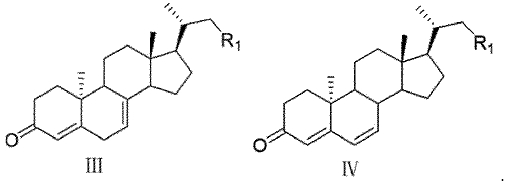

In some embodiments, the compound represented by structural formula IV is converted from the compound represented by structural formula III by subjecting the compound represented by structural formula III to a protonic acid condition to migrate the double bond from C-7,8 to C-6,7 to obtain the compound represented by structural formula IV.

Since the conversion rates are low when using hydrochloric acid, sulfuric acid, perchloric acid, glacial acetic acid, p-toluenesulfonic acid, trifluoroacetic acid, etc., in some embodiments, the protonic acid used may be HCl, HBr, etc.

Due to the particularity of the chemical structure, this step of reaction also has strict requirements on moisture content, acid concentration, and acid amount in order to obtain a high conversion rate. In some embodiments, the compound represented by structural formula IV is converted from the compound represented by structural formula III by adding the alcohol solution of hydrogen halide to a reaction solvent comprising the compound represented by structural formula III. Specifically, 10 mL-15 mL of the alcohol solution of hydrogen halide per 1 g of the compound represented by structural formula III is added, that is, the alcohol solution of hydrogen halide is added at an amount of 10v-15v. The mass percentage of water in the alcohol solution of hydrogen halide is less than 0.2%, and the weight of hydrogen halide accounts for 25wt%-40wt% of the total weight of the alcohol solution of hydrogen halide.

In the alcohol solution of hydrogen halide, ethanol, isopropanol, butanol, ethanediol, etc. can be used as the alcohol. The conversion rate can be as high as 89% when using ethanol, while the conversion rate is only about 55% when using methanol under the same conditions.

In some embodiments, an antioxidant with a mass fraction of 0.8%-1.2% (based on the mass of the compound represented by structural formula III) can also be added in this step to inhibit peroxidized impurities to improve the yield. Examples of antioxidants include sodium ascorbate and TBHQ.

In addition, the melting point of the compound represented by structural formula IV is low, making it difficult to obtain a solid, and it may deteriorate into oil to a certain extent under high-concentration acid, affecting the properties of the solid. In the embodiments of the present application, in order to avoid such phenomena, ethanol is used as the solvent during post-processing while stirring and gradient cooling to obtain a solid. The crude product is then beaten with n-heptane to remove oil to obtain a higher yield.

### (Steps of hydrolysis)

In some embodiments, the intermediate compound is compound E; the method further comprises hydrolyzing the compound represented by structural formula IV to obtain compound E;

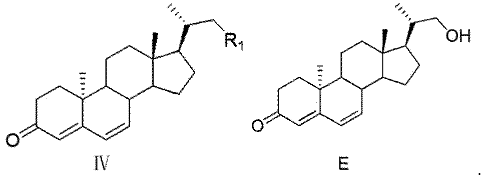

In some embodiments, R₁ is OR₃ in the compound represented by structural formula IV, wherein R₃ is 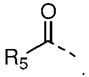 The compound represented by structural formula E is converted from the compound represented by structural formula IV by hydrolyzing the compound represented by structural formula IV under alkaline conditions to form a hydroxyl structure at C-21, where the alkaline substance used comprises at least one of NaOH, KOH, potassium acetate, sodium acetate and sodium benzoate.

In some embodiments, R₁ is OR₃ and R₃ is 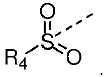 The compound represented by structural formula E is converted from the compound represented by structural formula IV by converting R₁ in the compound represented by structural formula IV into an ester group using DMF and KOAc, and then hydrolyzing under alkaline conditions, where the alkaline substance used comprises at least one of NaOH, KOH, potassium acetate, sodium acetate and sodium benzoate.

### Method for preparing dydrogesterone from intermediate

The embodiments of the present application also provide a method for preparing dydrogesterone, comprising constructing a ketone group at C-20 of the intermediate compound of the embodiments of the present application.

In some embodiments, the intermediate compound has the following structural formula:

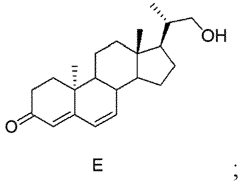

The ketone group at C-20 of the intermediate compound is constructed by:
oxidizing the hydroxyl group at C-21 of the intermediate compound into an aldehyde group to obtain compound F. The oxidation system may comprise NaClO, NaBr and 2,2,6,6-tetramethylpiperidin-1-oxyl radical, and the pH range of the oxidation system is controlled at 8-9; 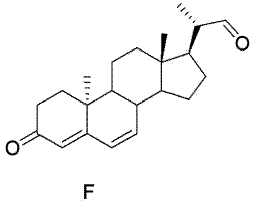
subjecting the aldehyde group of compound F to an enamination reaction to obtain compound Ga, wherein R₈ and R₉ are selected from a C1-C6 alkyl group, or R₈-N-R₉ form a 5- to 7-membered nitrogen heterocycle; for example, subjecting compound F and 1-(1-piperidyl) cyclohexene to an enamination reaction to obtain compound G; and 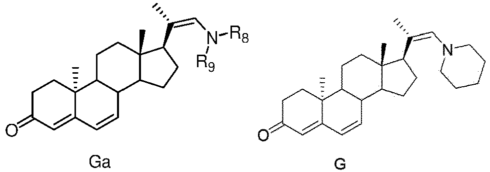
oxidizing compound G to obtain dydrogesterone; for example, oxidizing compound G in air under Cu⁺ catalysis to obtain dydrogesterone.

As an example, one of the synthetic routes L1 for preparing dydrogesterone H in the embodiments of the present application is as follows: 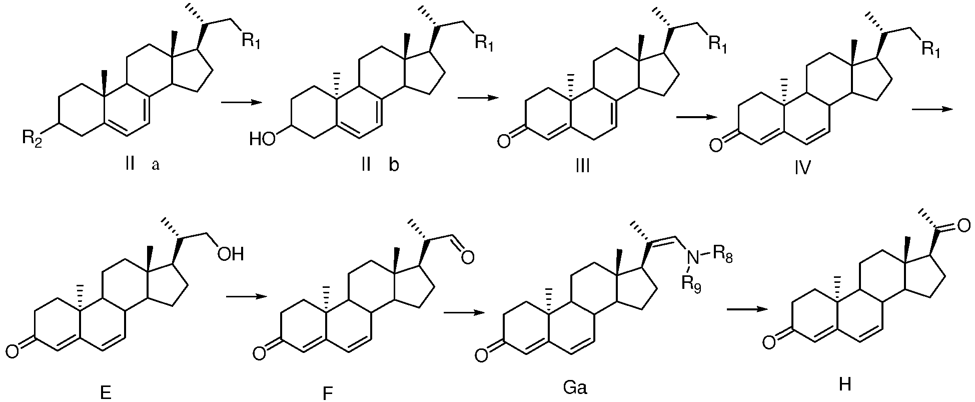 wherein R₂ is -OR₆.

It should be noted that in the above synthesis route L1, each arrow represents one step or several steps. For example, when R₂ is OH, IIb can be obtained directly through photoconversion. When R2 is another group, in addition to the photoconversion step, one or several steps (can be before or after photoconversion) are required to obtain IIb.

R₁ of different compounds in the above synthetic route L1 may be the same or different within the definition range. To formally reflect this, it can be converted into the following route:

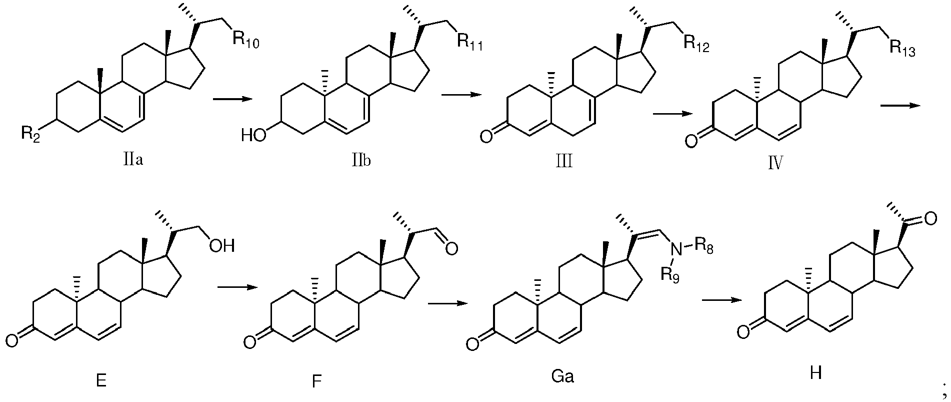

Wherein the definitions of R₁₀, R₁₁, R₁₂ and R₁₃ are the same as the above R1, and each is selected independently within the definition range. In some embodiments, R₁₀ is OR₃; R₁₁, R₁₂ and R₁₃ are halogen or OR₃; R₃ is 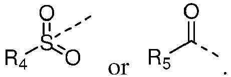 In some embodiments, R₁₀ is an OTs group. After photoconversion, OTs is converted into OAc or Br (at this time, OTs, OAc or Br are collectively referred to as Rn), and then the reaction from IIb to III is performed. In some embodiments, R₁₀, R₁₁ and R₁₂ are OTs groups. After obtaining the compound represented by formula IV, OTs is converted into OAc (at this time, OTs and OAc are collectively referred to as R₁₃), and then hydrolyzed.

When preparing dydrogesterone H according to the above synthetic route L1, the raw material used is the compound represented by structural formula IIa. However, in other embodiments, the compound represented by structural formula IIa may not be used as the starting material for preparing dydrogesterone, but compound E or any compound before compound E (compound represented by structural formula IIb, compound represented by structural formula III, or compound represented by structural formula IV) may be used as the starting material. Depending on the selected starting materials, the corresponding synthesis route can be adjusted accordingly based on synthesis route L1. As an example, when selecting a compound represented by structural formula III as a raw material, part of the route IIa → IIb → III can be deleted based on the synthesis route L1.

As an example, in the synthesis route L1, R₁ is OTs and R₂ is OH in the structural formula IIa, that is, a specific compound A in the compound represented by structural formula IIa is selected as the raw material to synthesize dydrogesterone H, and the synthesis route L2 is obtained:

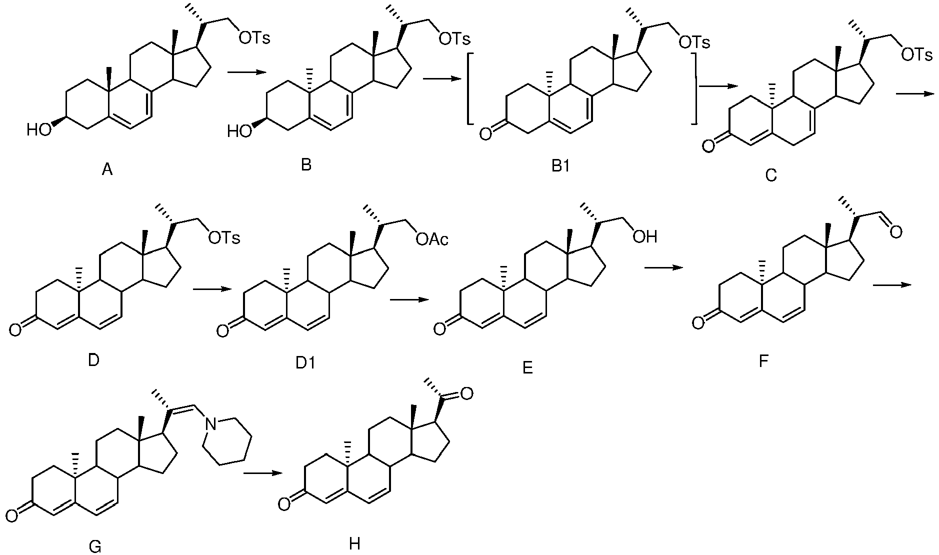

### Examples

Taking the synthetic route L2 as an example, the method for preparing dydrogesterone is illustrated through specific examples.

### Example 1: A→B

40 g of compound A and 500 mL of tetrahydrofuran were added to a photochemical reaction bottle, and irradiated for ring opening under ultraviolet light at 5-10°C for 8 h with a wavelength range of 270 nm-290 nm. When the raw materials and product monitored by HPLC presented about 70:20, irradiation under ultraviolet light was performed for another 8 h with a wavelength range of 300 nm-330 nm. When the raw materials and product monitored by HPLC presented about 55:35, irradiation was stopped. The organic phase was concentrated, replaced with methanol to a small volume, cooled down to -20°C, frozen for 1-2 h, filtered, and dried to obtain 16 g of white solid, which was mainly raw materials. The mother liquor was concentrated, replaced with acetonitrile to a small volume, cooled down to -20°C, frozen for 1-2 h, filtered, and dried to obtain 10 g of compound B as a white solid, with a primary yield of about 25%.

After detection, ¹H NMR (400 MHz, CDCl₃) δ 7.78 (d, *J* = 8.3 Hz, 2H), 7.34 (d, *J* = 8.1 Hz, 2H), 5.66-5.64 (m, 1H), 5.43 - 5.41 (m, 1H), 4.09 (s, 1H), 3.96 (dd, *J* = 9.3, 3.1 Hz, 1H), 3.83 (dd, *J* = 9.3, 6.0 Hz, 1H), 2.49-2.45 (m, 5H), 2.29-2.24 (m, 2H), 1.67 - 1.47 (m, 15H), 0.97 (d, *J* = 6.7 Hz, 3H), 0.72 (s, 3H), 0.57 (s, 3H).

### Example 1': A→B

40 g of compound A and 500 mL of tetrahydrofuran were added to a photochemical reaction bottle, and irradiated for ring opening under an ultraviolet high-pressure mercury lamp (500W) at 5-10°C for 8 h. During the ring opening, the light was filtered through an optical filter liquid (1 wt% copper chloride aqueous solution). When the raw materials and product monitored by HPLC presented about 70:20, irradiation was performed for another 8 h, during which the light was filtered through an optical filter liquid (2 wt% copper chloride concentration). When the raw materials and product monitored by HPLC presented about 55:35, irradiation was stopped. The organic phase was concentrated, replaced with methanol, cooled down to -20°C, frozen for 4 h, filtered, and dried to obtain 16 g of white solid, which was mainly raw materials. The mother liquor was concentrated, replaced with acetonitrile, cooled down to -20°C, frozen for 4 h, filtered, and dried to obtain 9.8 g of compound B as a white solid, with a primary yield of about 24.5%.

Comparative tests in Table 1 were carried out according to the above reaction steps, and other conditions were the same as in this example.

**Table 1 Experimental conditions and results**

| | Light filtering for ring opening | Light filtering for ring closing | Primary yield |
|---|---|---|---|
| 1 | No light filtering | No light filtering | 9% (product will not precipitate) |
| 2 | 1% copper chloride | 1% copper chloride | 20% |
| This example | 1% copper chloride | 2% copper chloride | 24.5% |

### Example 2: B→C

105 g (0.28 mol) of Dess-Martin periodinane (DMP) was added to a 1L three-necked flask, added with 3.78 g (0.21 mol) of water, 35 g (0.41 mol) of sodium bicarbonate and 500 mL of dichloromethane (DCM) while stirring, stirred for a while, added with 100 g (0.21 mol) of compound B at 5-10°C, and stirred for half an hour in this temperature. TLC showed that the raw material reacted and compound B1 was generated. The reaction solution was cooled down to -20°C, frozen for 1-2 h and filtered. The filter cake was rinsed with an appropriate amount of cold dichloromethane until there was no product in the filter cake. The organic phase was washed with sodium sulfite solution, sodium bicarbonate solution, and brine in sequence.

The organic phase was added with 100 mL of triethylamine and stirred at room temperature for 1-2 h. TLC showed that compound B1 was converted into compound C. The organic phase was washed with brine, 1M dilute hydrochloric acid, and brine in sequence. The organic phase was concentrated at 40°C, replaced with n-heptane to a small volume, cooled down to -20°C, frozen for 1-2 h, filtered, and dried to obtain 70 g of yellow solid, with a molar yield of about 70%.

1HNMR after detection: ¹H NMR (400 MHz, CDCl₃) δ 7.78 (d, *J* = 8.3 Hz, 2H), 7.34 (d, *J* = 8.1 Hz, 2H), 5.80 (s, 1H), 5.23 - 5.14 (m, 1H), 3.96 (dd, *J* = 9.3, 3.1 Hz, 1H), 3.83 (dd, *J* = 9.3, 6.0 Hz, 1H), 3.01 (ddd, *J* = 25.3, 22.6, 11.6 Hz, 2H), 2.56 - 2.27 (m, 6H), 2.21 (dd, *J* = 6.9, 2.6 Hz, 1H), 2.00 (dt, *J* = 13.4, 4.7 Hz, 1H), 1.93 - 1.11 (m, 13H), 1.04 (s, 3H), 0.97 (d, *J* = 6.7 Hz, 3H), 0.63 - 0.55 (m, 3H).

Comparative tests in Table 2 were carried out according to the above reaction steps, and other conditions were the same as in this example.

**Table 2 Experimental conditions and results**

| No. | Conditions | Conversion rate % | Yield % |
|---|---|---|---|
| 1 | 1.3eq DMP, DCM | 86 | 66 |
| 2 | 1.0eq DMP, DCM | 75 | 50 |
| 3 | 2.0eq DMP, DCM | 87 | 63 |
| 4 | 1.3eq DMP, chloroform | 85 | 62 |
| 5 | 1.3eq DMP, DCM, 2eq sodium bicarbonate | 90 | 68 |
| This example | 1.3eq DMP, DCM, 2eq sodium bicarbonate, 1eq water | 92 | 70 |

| | | | |
|---|---|---|---|
| Note: eq represents molar equivalent, the same below. | | | |

It can be seen from Table 2 that 1.3eq of Dess-Martin periodinane was enough. Increasing the amount did not significantly improve the conversion rate, but increased the difficulty of post-processing and affected the yield. Using chloroform as the solvent did not improve the yield. Adding sodium bicarbonate can promote the reaction and increase the conversion rate and yield. Adding a small amount of water can also promote the reaction and increase the conversion rate, yield and rate. The principle may be to form a more active DMP oxidation intermediate state in advance.

### Example 3: C→D

840 mL of absolute ethanol was added to a 1L three-necked flask, and introduced with dry hydrogen chloride gas at low temperature to prepare an absolute ethanol/hydrogen chloride solution (moisture content should be less than 0.2%, with a content of about 35%). To a 2L three-necked flask, 70 g (0.145 mol) of compound C, 700 mL of dichloromethane and 0.7 g of tert-butylhydroquinone (TBHQ) were added, dissolved to clarity, and protected under nitrogen. At 0°C-10°C, 840 mL of the homemade absolute ethanol/hydrogen chloride solution was added dropwise. The temperature was controlled for reaction of about 1 h. TLC detected that the remaining raw materials were less than 3%. Purified water was added to quench the reaction and liquid separation was performed. The organic phase was washed with sodium bicarbonate solution until PH=7-8. The organic phase was concentrated at below 50°C, replaced with ethanol while retaining about 500 mL of ethanol, and gradient cooled down

while stirring. A yellow solid precipitated, which was cooled down to -20°C, frozen for 1-2 h and filtered. The crude product was beaten with n-heptane, precipitated, filtered, and dried to obtain 50 g of off-white solid, with a molar yield of about 70%.

After detection, ¹H NMR (400 MHz, CDCl₃) δ 7.76 (d, *J* = 8.3 Hz, 2H), 7.33 (d, *J* = 8.1 Hz, 2H), 6.24 - 6.05 (m, 2H), 5.65 (s, 1H), 3.94 (dd, *J* = 9.3, 3.0 Hz, 1H), 3.80 (dd, *J* = 9.2, 6.0 Hz, 1H), 2.60 - 2.48 (m, 1H), 2.44 (s, 3H), 2.41 - 2.32 (m, 1H), 2.27 - 2.14 (m, 1H), 1.91 - 1.55 (m, 10H), 1.40 - 1.29 (m, 1H), 1.29 - 1.07 (m, 6H), 0.98 (d, *J* = 6.7 Hz, 3H), 0.70 (s, 3H).

¹³C NMR (101 MHz, CDCl₃) δ 199.59 (s), 163.38 (s), 144.65 (s), 140.96 (s), 132.91 (s), 129.75 (s), 127.85 (s), 126.81 (s), 123.60 (s), 75.47 (s), 51.57 (s), 49.22 (s), 42.70 (s), 39.59 (s), 38.65 (s), 38.24 (s), 37.10 (s), 36.07 (s), 35.49 (s), 33.89 (s), 27.00 (s), 24.89 (s), 22.14 (s), 21.59 (s), 20.46 (s), 16.72 (s), 10.58 (s).

Mass spectrum: C₂₉H₃₈O₄S, 482.9.

Comparative tests in Table 3 were carried out according to the above reaction steps, and other conditions were the same as in this example.

**Table 3 Experimental conditions and results**

| No. | Conditions | Conversion rate % | Yield % |
|---|---|---|---|
| 1 | Concentrated hydrochloric acid /Absolute ethanol | 20 | - |
| 2 | Concentrated sulfuric acid /Absolute ethanol | 30 | - |
| 3 | Trifluoroacetic acid /Absolute ethanol | 25 | - |
| 4 | Trifluoromethanesulfonic acid /Absolute ethanol | 40 | - |
| 5 | 12v HCl (35%)/Absolute ethanol | 89 | 67 |
| 6 | 12v HBr (35%)/Absolute ethanol | 87 | 65 |
| 7 | 12v HCl (20%)/Absolute ethanol | 70 | - |
| 8 | 5v HCl (35%)/Absolute ethanol | 50 | - |
| 9 | 20v HCl (35%)/Absolute ethanol | 90 | 60 |
| 10 | 12v HCl (35%)/Anhydrous methanol | 55 | - |
| 11 | 12v HCl (35%)/ Isopropanol | 85 | 62 |
| 12 | 12v HCl (35%)/ Anhydrous Tetrahydrofuran | 75 | - |
| 13 | 12v HCl (35%)/95% ethanol | 30 | - |
| 14 | 12v HCl (35%)/Absolute ethanol, 1% sodium ascorbate | 91 | 69 |
| This example | 12v HCl (35%)/Absolute ethanol, 1% TBHQ | 92 | 70 |

| | | | |
|---|---|---|---|
| Note: v represents the volume of solvent in mL required for each g of compound. For example, 12v HCl (35%)/absolute ethanol represents 12 mL HCl (35%)/absolute ethanol used per 1 g of compound. | | | |

As can be seen from Table 3, this reaction has strict requirements on moisture. The conversion rate of reactions using concentrated hydrochloric acid or 95% ethanol is low, and dry hydrogen chloride gas/absolute ethanol system is required to greatly increase the conversion rate. This reaction has requirements on the concentration of acid. When the acid content is low (20%), the conversion rate is relatively low. Better results can be achieved when the acid content is 30-38%. This reaction has requirements on the amount of acid, 10v-15v is more suitable. If it is too low, the conversion rate will be low, and if it is too high, it will lead to product degradation, which is manifested in that the solid is oily during post-processing and difficult to precipitate, resulting in reduced yield. In addition, the reaction is related to the strength of the acid, and the conversion rates of concentrated sulfuric acid, trifluoroacetic acid and trifluoromethanesulfonic acid are different. Interestingly, when anhydrous methanol is used as a solution, the conversion rate is also low. When tetrahydrofuran and isopropanol are used as solvents, the conversion rate is still lower than absolute ethanol. Adding antioxidants to the reaction can inhibit peroxidized impurities and increase the yield.

### Example 4: D→D1

To a 500 mL three-necked flask, 50 g (0.10 mol) of compound D and 200 mL of DMF were added, dissolved to clarity, added with 50 g (0.51 mol) of potassium acetate, and reacted at 100°C for 2 h. TLC detected that the raw material reacted completely. The reaction solution was slowly poured into 1L of water to precipitate a solid, stirred for 1 h, filtered and dried to obtain 36 g of yellow solid, with a molar yield of about 95%.

After detection, ¹H NMR (400 MHz, CDCl₃) δ 6.15 (dt, *J* = 19.5, 7.4 Hz, 2H), 5.64 (s, 1H), 4.07 (dd, *J* = 10.7, 3.5 Hz, 1H), 3.76 (dd, *J* = 10.7, 7.3 Hz, 1H), 2.59 - 2.44 (m, 1H), 2.40 (ddd, *J* = 11.5, 7.4, 4.9 Hz, 2H), 2.24 (ddd, *J* = 13.1, 5.2, 1.9 Hz, 1H), 2.03 (s, 3H), 1.95 - 1.67 (m, 7H), 1.67 - 1.52 (m, 2H), 1.43 - 1.31 (m, 2H), 1.29 - 1.13 (m, 5H), 1.00 (d, *J* = 6.6 Hz, 3H), 0.75 (s, 3H).

¹³C NMR (101 MHz, CDCl₃) δ 199.40 (s), 171.22 (s), 163.31 (s), 141.05 (s), 126.73 (s), 123.59 (s), 69.30 (s), 52.66 (s), 49.32 (s), 42.80 (s), 39.68 (s), 38.73 (s), 38.45 (s), 37.13 (s), 35.61 (d, *J* = 13.6 Hz), 33.90 (s), 27.21 (s), 25.01 (s), 22.14 (s), 20.91 (s), 20.53 (s), 16.99 (s), 10.66 (s).

Mass spectrum: C₂₄H₃₄O₃, 371.0.

### Example 5: D1→E

To a 250 mL three-necked flask, 36 g (0.10 mol) of compound D1 and 180 mL methanol were added, protected under nitrogen, cooled down to 0°C-5°C, added with 7.2 g (0.18 mol) of sodium hydroxide solid while controlling the temperature to less than 25°C, and then returned to room temperature naturally for 0.5-1 h of reaction. TLC monitored that the reaction was complete. Acetic acid was added to neutralize, and then 180 mL of water was slowly added dropwise to precipitate a solid, which was stirred in an ice bath for 1 h, filtered, and dried to obtain 30 g of yellow solid, with a molar yield of about 95%.

After detection, ¹H NMR (400 MHz, CDCl₃) δ 6.17 (dt, *J* = 23.1, 7.5 Hz, 2H), 5.65 (s, 1H), 3.64 (dd, *J* = 10.5, 3.2 Hz, 1H), 3.39 (dd, *J* = 10.5, 6.6 Hz, 1H), 2.69 - 2.46 (m, 1H), 2.46 - 2.32 (m, 2H), 2.25 (ddd, *J* = 13.1, 5.2, 1.9 Hz, 1H), 1.82 (dddd, *J* = 16.7, 14.9, 13.7, 9.7 Hz, 6H), 1.69 - 1.50 (m, 4H), 1.38 (ddd, *J* = 17.9, 12.4, 4.6 Hz, 2H), 1.28 - 1.15 (m, 5H), 1.05 (d, *J* = 6.6 Hz, 3H), 0.76 (s, 3H).

¹³C NMR (101 MHz, CDCl₃) δ 199.62 (s), 163.54 (s), 141.31 (s), 126.72 (s), 123.56 (s), 67.78 (s), 52.27 (s), 49.36 (s), 42.72 (s), 39.73 (s), 38.93 - 38.34 (m), 37.17 (s), 35.55 (s), 33.93 (s), 27.32 (s), 25.05 (s), 22.15 (s), 20.57 (s), 16.65 (s), 10.70 (s).

Mass spectrum: C₂₂H₃₂O₂, 329.0.

### Example 6: E→F→G→H

### (1)E→F

To a 250 mL three-necked flask, 30 g (91.3 mmol) of compound E and 150 mL of dichloromethane were added, then added with 1.5 g (9.6 mmol) of tempo, dissolved 1.08 g of sodium bromide (10.5 mmol) and 30 mL of 5% sodium bicarbonate aqueous solution while stirring, protected under nitrogen, cooled down to 0°C-5°C, and added with sodium hypochlorite dropwise. The temperature was controlled to less than 15°C for 0.5-1 h of reaction. TLC monitored that the reaction was completed. The reaction solution was quenched with sodium thiosulfate solution, stirred for 10 min, and subjected to liquid separation. The organic phase was washed once with brine, concentrated at below 50°C, replaced with petroleum ether while retaining 3v-5v petroleum ether, cooled down to 0°C for 2 h of precipitation, and filtered. The filter cake was rinsed with glacial petroleum ether, and dried to obtain 28 g of compound F as a solid, with a molar yield of about 92%.

¹H NMR (400 MHz, CDCl₃) δ 9.58 (d, *J* = 3.1 Hz, 1H), 6.30 - 6.04 (m, 2H), 5.67 (s, 1H), 2.53 (dd, *J* = 14.2, 5.4 Hz, 1H), 2.50 - 2.32 (m, 3H), 2.26 (ddd, *J* = 13.2, 5.3, 2.1 Hz, 1H), 1.98 - 1.78 (m, 5H), 1.73 - 1.37 (m, 6H), 1.36 - 1.22 (m, 4H), 1.13 (t, *J* = 6.1 Hz, 3H), 0.80 (s, 3H).

¹³C NMR (101 MHz, CDCl₃) δ 208.83 (s), 199.28 (s), 162.87 (s), 140.34 (s), 126.99 (s), 123.80 (s), 63.29 (s), 49.78 (s), 44.15 (s), 39.59 (s), 38.50 (s), 37.64 (s), 37.10 (s), 35.50 (s), 33.87 (s), 31.40 (s), 25.07 (s), 22.49 (s), 22.21 (s), 20.47 (s), 11.98 (s).

Mass spectrum: C₂₂H₃₀O₂, 327.0.

### (2) F→G→H (Dydrogesterone)

To a 100 mL three-necked flask, 28 g (85.8 mmol) of compound F and 42 mL anhydrous acetonitrile were added, then added with 22 g (122 mmol) of cyclohexene piperidine (content about 90%) while stirring, protected under nitrogen, stirred and dissolved to clarity at 40°C, added with glacial acetic acid for 3-6 h of reaction, cooled down to -20°C, precipitated for 2 h, and filtered. The filter cake was rinsed with glacial acetonitrile, drained, and dried in a vacuum drying oven at 35°C to obtain 28 g of compound G.

To a 100 mL three-necked flask, 0.42g (4.2 mmol) cuprous chloride and 42 mL DMF were added, replaced with nitrogen three times, heated to 65°C, stirred for 1 h at this temperature under nitrogen protection, and then cooled down to room temperature for later use. To a 500 mL three-necked flask, 28 g (71.2 mmol) of compound G and 280 mL of dichloromethane were added, cooled down to 0-5°C, added with cuprous chloride solution, and introduced with dry air for 4-8 h of reaction while maintaining the gas flow rate at 1L/min. TLC detected that the remaining raw material was less than 2%. If there was no change after extending the time, it can be stopped. The reaction solution was quenched with 10% sulfuric acid solution and subjected to liquid separation. The organic phase was washed with 1% sulfuric acid solution, added with 0.43 g of acetic acid, stirred for 5 min, then added with 6% sodium chlorite solution, and stirred at room temperature for 30 min. TLC showed that the raw materials were almost exhausted. The reaction solution was quenched with sodium thiosulfate and subjected to liquid separation. The organic phase was washed with 0.5% sodium hydroxide followed by brine, concentrated at below 50°C, replaced with water to obtain a crude product. The crude product was added with 280 mL of acetone, heated, dissolved to clarity, concentrated to a small volume, cooled down to -20°C, precipitated for 2 h, and filtered. The filter cake was rinsed with glacial acetone, drained, and dried in a 45°C oven to obtain 20 g of compound F as a solid, with a molar yield of about 74.6%.

After detection, ¹H NMR (400 MHz, CDCl₃) δ 9.56 (d, *J* = 3.1 Hz, 1H), 6.14 (dd, *J* = 10.8, 7.2 Hz, 2H), 5.65 (s, 1H), 2.59 - 2.44 (m, 1H), 2.44 - 2.29 (m, 3H), 2.25 (ddd, *J* = 13.1, 5.3, 1.9 Hz, 1H), 2.01 - 1.70 (m, 7H), 1.70 - 1.31 (m, 6H), 1.31 - 1.20 (m, 4H), 1.12 (d, *J* = 6.9 Hz, 3H), 0.78 (s, 3H).

¹³C NMR (101 MHz, CDCl₃) δ 204.51 (s), 199.36 (s), 163.08 (s), 140.68 (s), 126.90 (s), 123.70 (s), 50.79 (s), 49.34 (s), 48.93 (s), 43.21 (s), 39.72 (s), 38.58 (s), 38.32 (s), 37.10 (s), 35.51 (s), 33.88 (s), 26.66 (s), 25.27 (s), 22.13 (s), 20.47 (s), 13.28 (s), 11.00 (s).

Mass spectrum: C₂₁H₂₈O₂, 313.0.

The following examples provide other synthetic methods for some links in the synthetic route of dydrogesterone. The routes of Examples 7-9 are as follows:

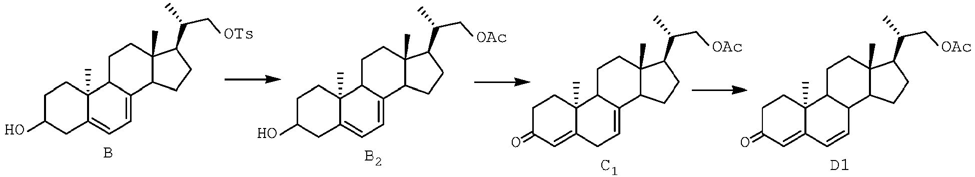

### Example 7

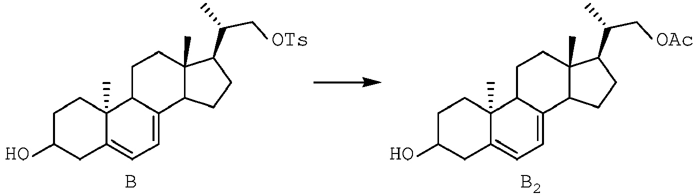

To a 500 mL three-necked flask, 50 g (0.10 mol) of compound B and 200 mL DMF were added, dissolved to clarity, added with 50 g (0.51 mol) of potassium acetate, and reacted at 100°C for 2 h. TLC detected that the raw material reacted completely. The reaction solution was slowly poured into 1L of water to precipitate a solid, stirred for 1 h, filtered and dried to obtain 35 g of yellow solid, with a molar yield of about 92%.

After detection, ¹H NMR (400 MHz, CDCl₃) δ 5.64 (dd, *J* = 5.3, 2.5 Hz, 1H), 5.52 - 5.39 (m, 1H), 4.06 (dd, *J* = 10.8, 3.4 Hz, 2H), 3.78 (dd, *J* = 10.7, 7.4 Hz, 1H), 2.59 - 2.39 (m, 2H), 2.25 (dt, *J* = 15.7, 2.6 Hz, 2H), 2.03 (s, 3H), 1.94 (dd, *J* = 8.6, 3.8 Hz, 2H), 1.75 - 1.59 (m, 7H), 1.42 (ddd, *J* = 29.8, 15.4, 6.9 Hz, 6H), 0.98 (d, *J* = 6.6 Hz, 3H), 0.71 (s, 3H), 0.61 (s, 3H).

### Example 8

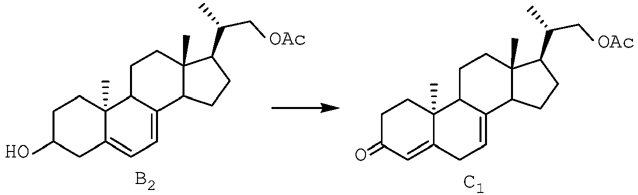

To a 1L three-necked flask, 78 g (0.21 mol) of compound B₂, 3.78 g of water (0.21 mol), 35 g of sodium bicarbonate (0.41 mol) and 500 mL of dichloromethane were added, and 105 g of Dess-Martin periodinane (0.28 mol) was added at 5-10°C and stirred for half an hour at this temperature. TLC showed that the raw material reacted and an intermediate isomer was generated. The reaction solution was cooled down to -20°C, frozen for 1-2 h and filtered. The filter cake was rinsed with an appropriate amount of cold dichloromethane until there was no product in the filter cake. The organic phase was washed with sodium sulfite solution, sodium bicarbonate solution, and brine in sequence.

The organic phase was added with 100 mL of triethylamine and stirred at room temperature for 1-2 h. TLC showed that the intermediate isomer was converted into compound C₁. The organic phase was washed with brine, 1M dilute hydrochloric acid, and brine in sequence. The organic phase was concentrated at 40°C, replaced with n-heptane to a small volume, cooled down to -20°C, frozen for 1-2 h, filtered, and dried to obtain 51 g of yellow solid, with a molar yield of about 65%.

After detection, ¹H NMR (400 MHz, CDCl₃) δ 5.79 (s, 1H), 5.25 - 5.16 (m, 1H), 4.05 (dd, *J*= 10.7, 3.4 Hz, 1H), 3.77 (dd, *J* = 10.7, 7.3 Hz, 1H), 3.62 - 3.54 (m, 1H), 3.15 - 2.86 (m, 2H), 2.44 - 2.36 (m, 1H), 2.25 - 2.17 (m, 1H), 2.03 (s, 3H), 1.87 - 1.67 (m, 7H), 1.54 - 1.49 (m, 2H), 1.38 (d, *J* = 11.2 Hz, 2H), 1.26 - 1.23 (m, 2H), 1.03 (s, 3H), 0.98 (d, *J* = 6.6 Hz, 3H), 0.65 (s, 3H).

### Example 9:

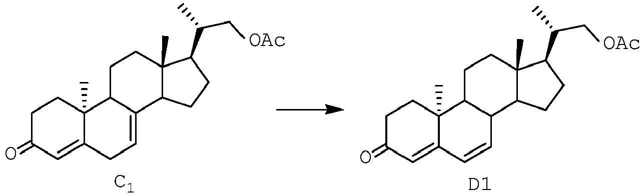

840 mL of absolute ethanol was added to a 1L three-necked flask, and introduced with dry hydrogen chloride gas at low temperature to prepare an absolute ethanol/hydrogen chloride solution (moisture content should be less than 0.2%, with a content of about 35%). To a 2L three-necked flask, 54 g (0.146 mol) of compound C₁, 0.54 g of TBHQ, and 700 mL of dichloromethane were added, dissolved to clarity, and protected under nitrogen. At 0°C-10°C, 840 mL of the homemade absolute ethanol/hydrogen chloride solution was added dropwise. The temperature was controlled for reaction of about 1 h. TLC detected that the remaining raw materials were less than 3%. Purified water was added to quench the reaction and liquid separation was performed. The organic phase was washed with sodium bicarbonate solution until PH=7-8. The organic phase was concentrated at below 50°C, replaced with ethanol while retaining about 500 mL of ethanol, and gradient cooled down while stirring. A yellow solid precipitated, which was cooled down to -20°C, frozen for 1-2 h and filtered. The crude product was beaten with n-heptane, precipitated, filtered, and dried to obtain 36 g of off-white solid, with a molar yield of about 66.7%

After detection, ¹H NMR (400 MHz, CDCl₃) δ 6.15 (dt, *J* = 19.5, 7.4 Hz, 2H), 5.64 (s, 1H), 4.07 (dd, *J* = 10.7, 3.5 Hz, 1H), 3.76 (dd, *J* = 10.7, 7.3 Hz, 1H), 2.59 - 2.44 (m, 1H), 2.40 (ddd, *J* = 11.5, 7.4, 4.9 Hz, 2H), 2.24 (ddd, *J* = 13.1, 5.2, 1.9 Hz, 1H), 2.03 (s, 3H), 1.95 - 1.67 (m, 7H), 1.67 - 1.52 (m, 2H), 1.43 - 1.31 (m, 2H), 1.29 - 1.13 (m, 5H), 1.00 (d, *J =* 6.6 Hz, 3H), 0.75 (s, 3H).

¹³C NMR (101 MHz, CDCl₃) δ 199.40 (s), 171.22 (s), 163.31 (s), 141.05 (s), 126.73 (s), 123.59 (s), 69.30 (s), 52.66 (s), 49.32 (s), 42.80 (s), 39.68 (s), 38.73 (s), 38.45 (s), 37.13 (s), 35.61 (d, *J* = 13.6 Hz), 33.90 (s), 27.21 (s), 25.01 (s), 22.14 (s), 20.91 (s), 20.53 (s), 16.99 (s), 10.66 (s).

Mass spectrum: C₂₄H₃₄O₃, 371.0.

### The route of Example 10-13 is as follows:

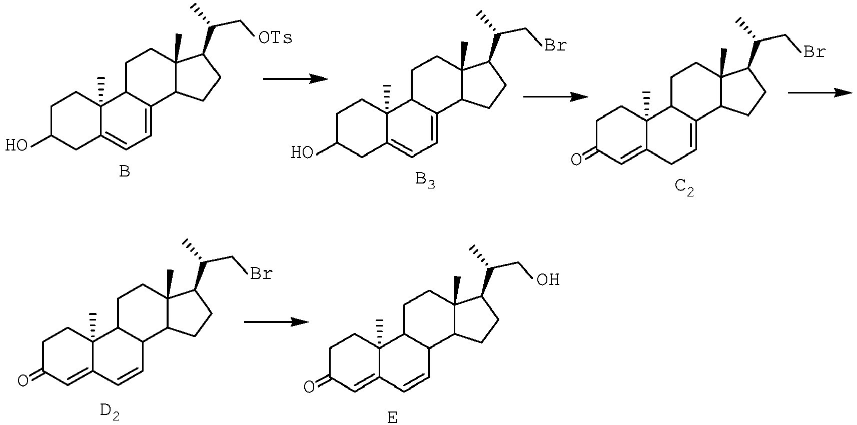

### Example 10:

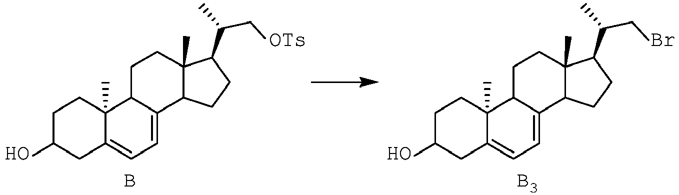

To a 500 mL three-necked flask, 50 g (0.10 mol) of compound B and 200 mL DMF were added, dissolved to clarity, added with 17.4 g (0.21 mol) of lithium bromide, and reacted at 100°C for 2 h. TLC detected that the raw material reacted completely. The reaction solution was slowly poured into 1L of water to precipitate a solid, stirred for 1 h, filtered and dried to obtain 37.7 g of yellow solid, with a molar yield of about 93%.

### Example 11:

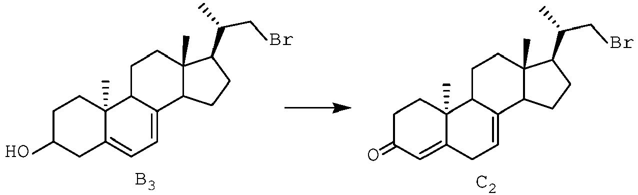

To a 1L three-necked flask, 82 g (0.21 mol) of compound B₃, 3.78 g of water (0.21 mol), 35 g of sodium bicarbonate (0.41 mol) and 500 mL of dichloromethane were added, and 105 g of Dess-Martin periodinane (0.28 mol) was added at 5-10°C and stirred for half an hour at this temperature. TLC showed that the raw material reacted and an intermediate isomer was generated. The reaction solution was cooled down to -20°C, frozen for 1-2 h and filtered. The filter cake was rinsed with an appropriate amount of cold dichloromethane until there was no product in the filter cake. The organic phase was washed with sodium sulfite solution, sodium bicarbonate solution, and brine in sequence.

The organic phase was added with 100 mL of triethylamine and stirred at room temperature for 1-2 h. TLC showed that the intermediate isomer was converted into compound BBr. The organic phase was washed with brine, 1M dilute hydrochloric acid, and brine in sequence. The organic phase was concentrated at 40°C, replaced with n-heptane to a small volume, cooled down to -20°C, frozen for 1-2 h, filtered, and dried to obtain 55.8 g of yellow solid, with a molar yield of about 68%.

### Example 12:

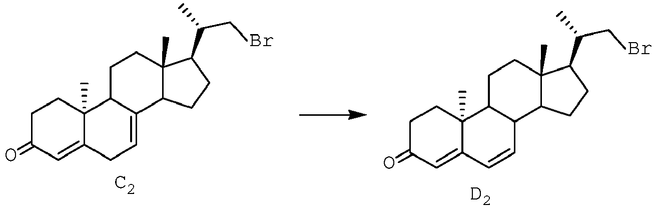

840 mL of absolute ethanol was added to a 1L three-necked flask, and introduced with dry hydrogen chloride gas at low temperature to prepare an absolute ethanol/hydrogen chloride solution (moisture content should be less than 0.2%, with a content of about 35%). To a 2L three-necked flask, 57 g (0.146 mol) of compound C₂, 0.57 g of TBHQ, and 700 mL of dichloromethane were added, dissolved to clarity, and protected under nitrogen. At 0°C-10°C, 840 mL of the homemade absolute ethanol/hydrogen chloride solution was added dropwise. The temperature was controlled for reaction of about 1 h. TLC detected that the remaining raw materials were less than 3%. Purified water was added to quench the reaction and liquid separation was performed. The organic phase was washed with sodium bicarbonate solution until PH=7-8. The organic phase was concentrated at below 50°C, replaced with ethanol while retaining about 500 mL of ethanol, and gradient cooled down while stirring. A yellow solid precipitated, which was cooled down to -20°C, frozen for 1-2 h and filtered. The crude product was beaten with n-heptane, precipitated, filtered, and dried to obtain 38 g of a solid, with a molar yield of about 66.7%.

After detection, ¹H NMR (400 MHz, CDCl₃) δ 6.30 - 6.04 (m, 2H), 5.66 (s, 1H), 3.50 (dd, J = 9.8, 2.5 Hz, 1H), 3.36 (dd, J = 9.8, 5.7 Hz, 1H), 2.64 - 2.48 (m, 1H), 2.47 - 2.34 (m, 2H), 2.32 - 2.21 (m, 1H), 1.95 - 1.55 (m, 9H), 1.47 - 1.17 (m, 7H), 1.09 (d, J = 6.5 Hz, 3H), 0.77 (s, 3H).

### Example 13:

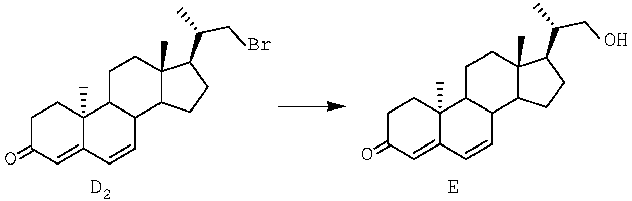

To a 250 mL three-necked flask, 90 mL of methanol, 40 mL of dichloromethane and 30 g (76.6 mol) of compound D₂ were added, then added with 61 mL of sodium hydroxide aqueous solution (10%, 153.2 mmol) dropwise at 0°C to 10°C, and reacted at this temperature for about 1 h. TLC detected that the raw materials reacted completely. 10 mL acetic acid was added to quench the reaction. The organic phase was concentrated below 50°C to remove dichloromethane, replaced with methanol water, precipitated, filtered, and dried to obtain 22.7 g of a solid, with a molar yield of about 90%.

After detection, ¹H NMR (400 MHz, CDCl₃) δ 6.17 (dt, *J* = 23.1, 7.5 Hz, 2H), 5.65 (s, 1H), 3.64 (dd, *J* = 10.5, 3.2 Hz, 1H), 3.39 (dd, *J* = 10.5, 6.6 Hz, 1H), 2.69 - 2.46 (m, 1H), 2.46 - 2.32 (m, 2H), 2.25 (ddd, *J* = 13.1, 5.2, 1.9 Hz, 1H), 1.82 (dddd, *J* = 16.7, 14.9, 13.7, 9.7 Hz, 6H), 1.69 - 1.50 (m, 4H), 1.38 (ddd, *J* = 17.9, 12.4, 4.6 Hz, 2H), 1.28 - 1.15 (m, 5H), 1.05 (d, *J* = 6.6 Hz, 3H), 0.76 (s, 3H).

¹³C NMR (101 MHz, CDCl₃) δ 199.62 (s), 163.54 (s), 141.31 (s), 126.72 (s), 123.56 (s), 67.78 (s), 52.27 (s), 49.36 (s), 42.72 (s), 39.73 (s), 38.93 - 38.34 (m), 37.17 (s), 35.55 (s), 33.93 (s), 27.32 (s), 25.05 (s), 22.15 (s), 20.57 (s), 16.65 (s), 10.70 (s)

Mass spectrum: C₂₂H₃₂O₂, 329.0.

### Example 14:

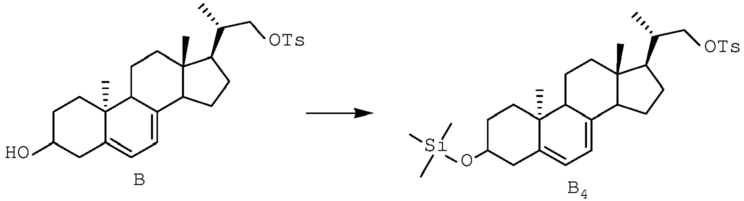

To a 500 mL three-necked flask, 200 mL of dichloromethane, 50 g (0.10 mol) of compound B, and 13.6 g (0.20 mol) of imidazole were added, then added with 16.3 g of trimethylchlorosilane (0.15 mol) dropwise at -5°C-5°C, and reacted at this temperature for about 1 h. TLC detected that the raw materials reacted completely. 10 mL water was added to quench the reaction. The organic phase was washed with water, dilute hydrochloric acid aqueous solution, and sodium bicarbonate solution in sequence. The organic phase was concentrated at below 50°C to remove dichloromethane, replaced with ethanol, precipitated, filtered, and dried to obtain 51.7 g of a solid, with a molar yield of about 90%.

B₄ was esterified using a method similar to Example 7, and then deprotected to obtain B₂.

## Claims

1. An intermediate compound, having a structural formula of 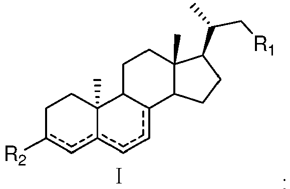
wherein, R₁ is halogen or OR₃;
R₂ is selected from the group consisting of =O, -OR₆, and a protected carbonyl group;
R₃ is selected from the group consisting of H, indicates a connection position with O; 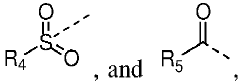 wherein dotted line
R₄ is selected from the group consisting of a substituted or unsubstituted C1-C6 straight or branched chain alkyl group, a phenyl group unsubstituted or substituted by a C1-C6 alkyl group, a hydroxyl group or a halogen atom, a naphthyl group unsubstituted or substituted by a C1-C6 alkyl group, a hydroxyl group or a halogen atom, and a pyridyl group unsubstituted or substituted by a C1-C6 alkyl group, a hydroxyl group or a halogen atom;
R₅ is selected from the group consisting of a substituted or unsubstituted C1-C6 straight or branched chain alkyl group, a phenyl group unsubstituted or substituted by a C1-C6 alkyl group, a hydroxyl group or a halogen atom, a naphthyl group unsubstituted or substituted by a C1-C6 alkyl group, a hydroxyl group or a halogen atom, and a pyridyl group unsubstituted or substituted by a C1-C6 alkyl group, a hydroxyl group or a halogen atom;
R₆ is H or a hydroxyl protecting group; and
in structural formula I, - - - - - - means that the chemical bond is a single bond or a double bond, and when one - - - - - - is a double bond, the adjacent one - - - - - - is a single bond.

2. The intermediate compound according to claim 1, wherein R₄ is a C1-C3 straight or branched chain alkyl group, or a phenyl group unsubstituted or substituted by a C1-C3 alkyl group; and/or
R₅ is selected from the group consisting of a C1-C3 straight or branched chain alkyl group, a phenyl group unsubstituted or substituted by a C1-C3 alkyl group, a naphthyl group unsubstituted or substituted by a C1-C3 alkyl group, and a pyridyl group unsubstituted or substituted by a C1-C3 alkyl group; and/or
the hydroxyl protecting group is selected from the group consisting of -C(=O)R₇, a C1-C8 alkyl group, and a C1-C8 silyl group; and/or
R₇ is a substituted or unsubstituted C1-C6 straight or branched alkyl group; and/or
the protected carbonyl group is a ketal; and/or
the halogen or the halogen atom is selected from the group consisting of Cl, Br, and I.

3. The intermediate compound according to claim 1, wherein R₃ is selected from the group consisting of H,

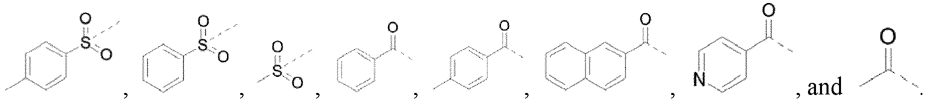

4. The intermediate compound according to any one of claims 1 to 3, wherein the intermediate compound comprises a structural formula selected from the group consisting of: 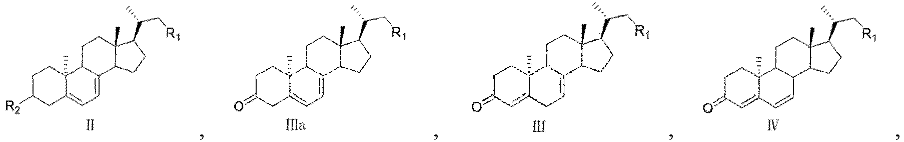 and 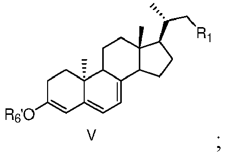 wherein, R₂ is -OR₆ or a protected carbonyl group, and R₆' is a C1-C8 alkyl group.

5. The intermediate compound according to claim 4, wherein the intermediate compound comprises a structural formula selected from the group consisting of: 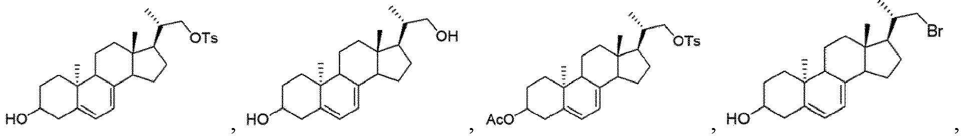 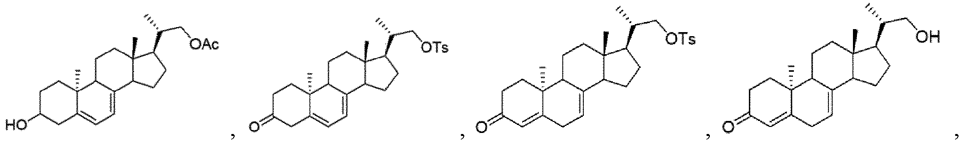 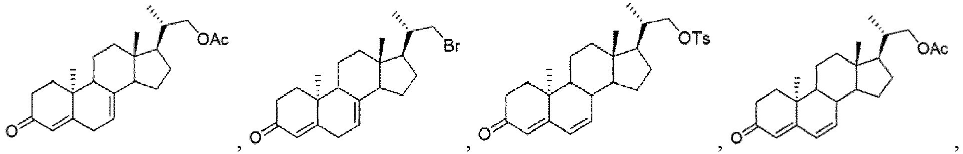 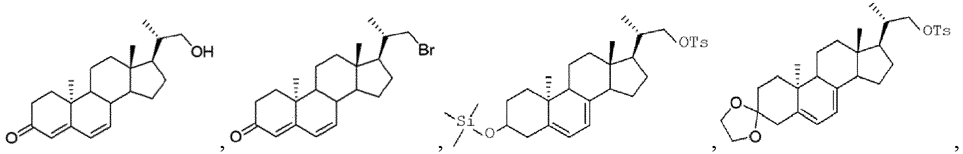 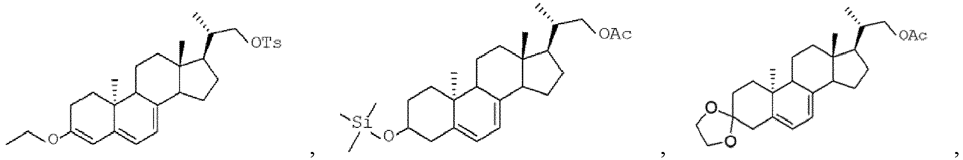 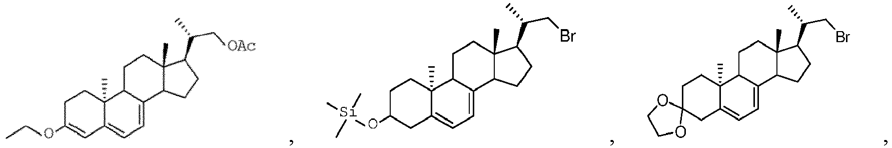 and

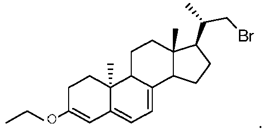

6. A method for preparing the intermediate compound according to any one of claims 1 to 5, comprising subjecting a compound represented by structural formula IIa to photochemical conversion to switch the methyl group at C-10 from β-configuration to α-configuration to obtain a compound represented by structural formula II;

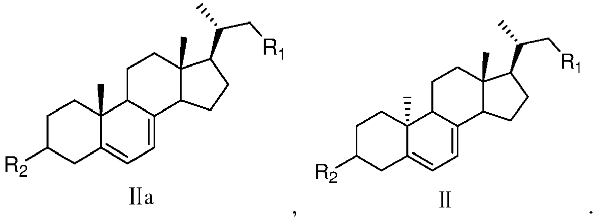

7. The method according to claim 6, comprising:
subjecting the compound represented by structural formula IIa to photochemical conversion under the irradiation of an ultraviolet high-pressure mercury lamp to switch the methyl group at C-10 from β-configuration to α-configuration;
wherein ultraviolet light emitted by the ultraviolet high-pressure mercury lamp is filtered by an optical filter liquid for photochemical conversion, and the optical filter liquid comprises Cu²⁺;
wherein the photochemical conversion is carried out using an optical filter liquid of a first stage and an optical filter liquid of a second stage, and a concentration of Cu²⁺ in the optical filter liquid of the first stage is less than or equal to a concentration of Cu²⁺ in the optical filter liquid of the second stage.

8. The method according to claim 7, wherein the concentration of Cu²⁺ in the optical filter liquid of the first stage is 0.1-0.5 wt%, and the concentration of Cu²⁺ in the optical filter liquid of the second stage is 0.5-1.2 wt%.

9. The method according to claim 6, wherein R₂ in the compound represented by structural formula IIa is -OR₆; and the compound represented by structural formula II is a compound represented by structural formula IIb;

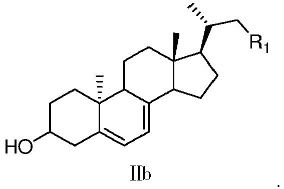

10. The method according to claim 9, wherein the method further comprises subjecting the compound represented by structural formula IIb to oxidation of the hydroxyl group at C-3 and double bond migration at C-5,6 to obtain a compound represented by structural formula III;

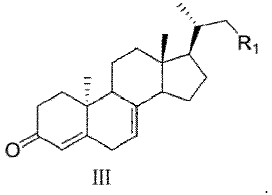

11. The method according to claim 10, wherein the compound represented by structural formula III is converted from the compound represented by structural formula IIb by:
performing an Oppenauer oxidation reaction to oxidize the hydroxyl group at C-3 to a ketone group and to migrate the double bond from C-5,6 to C-4,5; or
performing oxidation treatment on the compound represented by structural formula IIb by using an oxidizing reagent to oxidize the hydroxyl group at C-3 in the compound represented by structural formula IIb to a ketone group, wherein the oxidizing reagent has a structural formula of 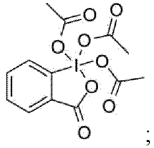 and performing alkaline treatment to migrate the double bond from C-5,6 to C-4,5 to obtain the compound represented by structural formula III.

12. The method according to claim 11, wherein bicarbonate and water are added during the oxidation treatment, and the bicarbonate, the water and the compound represented by structural formula IIb are at a molar ratio of (1.5-2.5):(0.8-1.2): 1.

13. The method according to claim 11, wherein an organic amine is employed for the alkaline treatment.

14. The method according to any one of claims 10 to 13, wherein the method further comprises subjecting the compound represented by structural formula III to double bond migration to obtain a compound represented by structural formula IV;

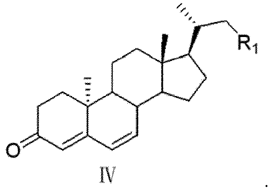

15. The method according to claim 14, wherein the compound represented by structural formula IV is converted from the compound represented by structural formula III by:
subjecting the compound represented by structural formula III to a protonic acid condition to migrate the double bond from C-7,8 to C-6,7 to obtain the compound represented by structural formula IV;
wherein the protonic acid is added in the form of an alcohol solution of hydrogen halide, and the alcohol is selected from the group consisting of ethanol, isopropanol, butanol, ethanediol, and a mixture thereof.

16. The method according to claim 15, wherein the compound represented by structural formula IV is converted from the compound represented by structural formula III by:
adding the alcohol solution of hydrogen halide to a reaction solvent comprising the compound represented by structural formula III;
wherein the alcohol solution of hydrogen halide is added at an amount of 10v-15v, a mass percentage of water in the alcohol solution of hydrogen halide is less than 0.2%, and a weight of hydrogen halide accounts for 25wt%-40wt% of a total weight of the alcohol solution of hydrogen halide.

17. The method according to claim 16, wherein the method further comprises adding an antioxidant to the reaction solvent comprising the compound represented by structural formula III, and a mass of the antioxidant accounts for 0.8%-1.2% of a mass of the compound represented by structural formula III.

18. The method according to claim 14, wherein the intermediate compound is compound E; the method further comprises hydrolyzing the compound represented by structural formula IV to obtain compound E; 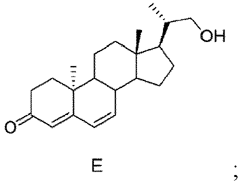 and R₁ is halogen or OR₃ in the compound represented by structural formula IV, wherein R₃ is

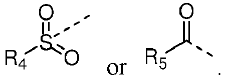

19. Application of the intermediate compound according to any one of claims 1 to 5 in the manufacture of dydrogesterone by constructing a ketone group at C-20 of the intermediate compound.

20. The application according to claim 19, wherein the intermediate compound has the following structural formula or the intermediate compound is converted into an intermediate compound with the following structure: 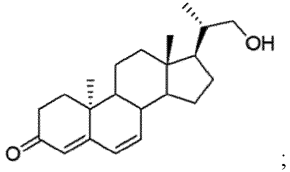 and the ketone group is constructed by:
oxidizing the hydroxyl group at C-21 of the intermediate compound into an aldehyde group to obtain compound F; and
subjecting the aldehyde group of compound F to an enamination reaction and then to oxidization at C-20 into a carbonyl group to obtain dydrogesterone;
